(19) Europäisches Patentamt
European Patent Office
Office européen des brevets

(11) **EP 3 692 595 B1**

(12) **FASCICULE DE BREVET EUROPEEN**

(45) Date de publication et mention
de la délivrance du brevet:
**04.09.2024 Bulletin 2024/36**

(21) Numéro de dépôt: **18796717.9**

(22) Date de dépôt: **04.10.2018**

(51) Classification Internationale des Brevets (IPC):
**H01Q 1/27** (2006.01)    **A61J 3/00** (2006.01)
**H01Q 1/38** (2006.01)    **H01Q 9/04** (2006.01)

(52) Classification Coopérative des Brevets (CPC):
**H01Q 1/273; H01Q 1/38; H01Q 9/0421;
H01Q 9/0442**

(86) Numéro de dépôt international:
**PCT/FR2018/052442**

(87) Numéro de publication internationale:
**WO 2019/069024 (11.04.2019 Gazette 2019/15)**

(54) **ANTENNE RADIOÉLECTRIQUE ROBUSTE EN IMPÉDANCE ET À BAS PROFIL**

**FLACHE IMPEDANZROBUSTE FUNKANTENNE**

**LOW-PROFILE, IMPEDANCE-ROBUST RADIO ANTENNA**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **04.10.2017 FR 1759267**

(43) Date de publication de la demande:
**12.08.2020 Bulletin 2020/33**

(73) Titulaires:
• **Bodycap**
**14200 Herouville Saint Clair (FR)**
• **Centre National de la Recherche Scientifique
(CNRS)**
**75016 Paris (FR)**
• **Université de Rennes 1**
**35065 Rennes Cedex (FR)**

(72) Inventeurs:
• **NIKOLAYEV, Denys**
**35700 Rennes (FR)**
• **ZHADOBOV, Maxim**
**35830 Betton (FR)**
• **SAULEAU, Ronan**
**35690 Acigné (FR)**

(74) Mandataire: **Fidal Innovation**
**4-6 avenue d'Alsace**
**92400 Courbevoie (FR)**

(56) Documents cités:
WO-A1-2011/111008    WO-A1-2017/086633
CN-A- 105 870 602    KR-A- 20140 119 606
US-A1- 2010 149 042    US-A1- 2017 117 620

## Description

DOMAINE TECHNIQUE

La présente description concerne une antenne radioélectrique et un dispositif biotélémétrique équipé d'une telle antenne radioélectrique.

ETAT DE L'ART

Dans le domaine des applications médicales, les dispositifs biotélémétriques sont utilisés pour l'acquisition de signaux physiologiques et l'analyse des données physiologiques associées.

Parmi les dispositifs biotélémétriques, on trouve des dispositifs biotélémétriques ingestibles et/ou implantables *in vivo,* qui peuvent servir à la fois à la collecte de signaux physiologiques et la mise en oeuvre de fonctions thérapeutiques, comme par exemple la délivrance de médicaments ou la stimulation électrique. Ces dispositifs biotélémétriques se présentent par exemple sous la forme de capsules ingestibles ou d'implants pouvant être insérés dans le corps d'humains ou d'animaux.

Ces dispositifs biotélémétriques intègrent une antenne radioélectrique configurée pour émettre et/ou recevoir une onde électromagnétique. L'antenne radioélectrique est utilisée pour transmettre des données vers un équipement extérieur de contrôle / d'analyse des données ou pour recevoir les instructions émis par cet équipement. Ces équipements externes sont utilisés notamment pour analyser *ex vivo* les données transmises par l'antenne radioélectrique.

Lorsque le dispositif biotélémétrique est placé dans un milieu environnant (par exemple un milieu biologique), l'antenne est fortement couplée avec ce milieu environnant et l'impédance de l'antenne radioélectrique est ainsi fortement dépendante des propriétés électromagnétiques (permittivité, conductivité) de ce milieu environnant. En particulier, une désadaptation de l'antenne radioélectrique par rapport au microcontrôleur peut se produire du fait des propriétés électromagnétiques du milieu environnant.

La demande de brevet US 2017/0117620 A1 divulgue une antenne comprenant un élément émetteur d'impédance ajustable incorporé dans le matériau d'un substrat d'une carte imprimée multicouches de telle sorte que l'élément émetteur soit formé de plusieurs éléments, répartis sur des couches de substrat différentes.

La demande de brevet KR 2014 0119606A divulgue une antenne capsule comprenant un substrat, un résonateur de structure hélicoïdale et un plan de masse formés sur une même face du substrat.

La demande de brevet internationale WO2017/086633 porte sur une antenne PIFA multi-bande implantable in vivo. L'antenne comprend deux plans de masse et un substrat, ainsi qu'un premier élément conducteur en forme de bande formé sur une première face du substrat et connecté à l'alimentation électrique. Un deuxième élément rectiligne conducteur peut être formé dans le premier plan de masse et ainsi placé en contact avec la seconde face du substrat. Cet élément rectiligne conducteur est connecté par un via au premier élément.

La demande de brevet WO2010/119207A1 divulgue un exemple d'antenne radioélectrique, comprenant un résonateur sous forme de corps plat, électriquement conducteur, comprenant deux éléments d'impédances caractéristiques distinctes, un premier élément étant formé par une bande de matériau conducteur et le deuxième élément étant formé par un serpentin avec un ou plusieurs coudes. Dans cet exemple, les deux éléments du corps plat sont de type microruban proches au milieu environnant. Les impédances caractéristiques de ces deux éléments sont donc susceptibles d'être affectées par les propriétés électromagnétiques variables du milieu environnant dans lequel cette antenne radioélectrique est utilisée.

Une telle antenne radioélectrique présente ainsi un fort couplage avec le milieu environnant et son impédance est donc affectée de manière sensible par le milieu environnant. Une telle antenne radioélectrique manque donc de polyvalence en ce que lorsque l'antenne radioélectrique est conçue pour un milieu environnant spécifique, elle peut mal fonctionner lorsqu'elle se trouve dans un autre milieu environnant. Ceci est particulièrement gênant dans le cadre de l'utilisation de ces antennes radioélectriques dans un dispositif biotélémétrique ingestibles et/ou implantables *in vivo* destiné à être en contact avec des milieux biologiques environnants variés.

Une désadaptation d'impédance entre l'antenne radioélectrique et le microprocesseur induit en outre des pertes d'énergie dans le dispositif biotélémétrique et impacte les performances en transmission de l'antenne radioélectrique, notamment la puissance de l'onde électromagnétique émis. Ceci est d'autant plus problématique que l'onde électromagnétique doit, pour parvenir à l'équipement externe, traverser le corps dans lequel le dispositif biotélémétrique a été introduit, et ce, avec des épaisseurs et natures de tissus à traverser pouvant être variables selon la localisation dans le corps du dispositif biotélémétrique.

Il apparaît ainsi un besoin de disposer d'une antenne radioélectrique robuste en impédance et peu sensible aux variations de propriétés électromagnétiques du milieu environnant, qui soit utilisable dans un dispositif biotélémétrique ingestible et/ou implantable *in vivo,* destiné à traverser des milieux biologiques aux propriétés électromagnétiques variées.

RESUME

La présente description a pour objet, selon un premier aspect, une antenne radioélectrique. Cette antenne radioélectrique comprend : un substrat formé en un matériau diélectrique ; un plan de masse en matériau électriquement conducteur, agencé sur une première face du substrat ; un résonateur configuré pour convertir un signal électrique incident en une onde électromagnétique, le résonateur comprenant un premier élément ayant une première impédance caractéristique et un deuxième élément ayant une deuxième impédance caractéristique supérieure à la première impédance caractéristique. Le premier élément est configuré pour recevoir le signal électrique incident, le premier élément est formé par une bande en matériau électriquement conducteur, la bande étant agencée sur une deuxième face du substrat opposée à la première face. Le deuxième élément est formé par un segment rectiligne, découpé dans le plan de masse et séparé du reste du plan de masse par une fente de largeur fixe. Le deuxième élément est relié électriquement au plan de masse au niveau d'une première extrémité du segment et relié électriquement au premier élément au niveau d'une deuxième extrémité du segment au moyen d'un via traversant le substrat. L'antenne est destinée à être placée de sorte que la deuxième face sur laquelle est arrangée le premier élément soit placée du côté d'un milieu environnant l'antenne et que le deuxième élément soit placé du côté opposé à ce milieu environnant.

L'antenne radioélectrique selon le premier aspect comprend à la fois un élément coplanaire, dans le plan de masse, et un élément sous forme bande de matériau électriquement conducteur (réalisé par exemple, selon la technologie dite de microruban).

Du fait que le deuxième élément soit formé par un segment rectiligne, la circulation des courants électriques dans cet élément est canalisée selon la direction de ce segment, ce qui évite des pertes liés à l'impureté de polarisation.

Une telle antenne radioélectrique est robuste en impédance du fait également de l'utilisation combinée d'une technologie microruban et d'une technologie coplanaire.

Du fait des deux faces de l'antenne radioélectrique, on peut placer l'antenne radioélectrique de sorte que la première face comprenant le premier élément soit du côté d'un milieu environnant et que le deuxième élément soit du côté opposé au milieu environnant. La variation des propriétés électromagnétiques du milieu environnant impactera principalement l'impédance caractéristique du premier élément. En outre, le deuxième élément à plus haute impédance caractéristique, est mieux découplé et isolé de ce milieu environnant, car ce deuxième élément est réalisé comme une ligne coplanaire dans le plan de masse de l'antenne.

Dans un ou plusieurs modes de réalisation, la largeur de la fente entre le segment rectiligne et le plan de masse est comparable ou inférieure à la distance entre le segment rectiligne et le milieu environnant, ce qui permet de « contraindre » le champ électrique non-propageant à l'intérieur du substrat et donc de réduire la sensibilité du deuxième élément aux variations des propriétés électromagnétiques du milieu environnant. Ainsi l'impédance caractéristique de l'antenne radioélectrique sera peu affectée par le milieu environnant ou le changement de milieu environnant ou aux variations des différentes propriétés électromagnétiques du milieu environnant, car l'effet sur l'impédance de l'antenne radioélectrique sera limité à l'effet sur l'impédance caractéristique du premier élément. Tout ceci contribue à augmenter la robustesse en impédance de l'antenne radioélectrique et à la réduction des pertes par défaut d'adaptation d'impédance entre l'antenne radioélectrique et le reste du circuit électronique dans le dispositif biotélémétrique. L'impédance de l'antenne est peu sensible aux variations du milieu environnant. Le coefficient de réflexion $S_{11}$, déterminé comme le rapport complexe entre l'intensité complexe du signal électrique incident converti par l'antenne en onde électromagnétique et l'intensité complexe du signal électrique réfléchi résultant d'une réflexion d'une fraction du signal électrique incident, est tel que $|S_{11}| < -10$ dB dans toute une gamme de milieux environnants.

On peut donc utiliser une telle antenne radioélectrique pour la réalisation d'un dispositif biotélémétrique ingestible et/ou implantable *in vivo* destiné à être en contact avec des milieux biologiques environnants variés (par exemple, tissus corporels humains et/ou animaux) et/ou pour de multiples scénarios d'application.

Une telle antenne se prête à une réalisation dans un circuit imprimé et peut en outre être intégrée dans une capsule biotélémétrique.

Une telle antenne radioélectrique se prête également à une réalisation d'antenne radioélectrique miniature, par exemple d'épaisseur de substrat inférieure à 1 mm et de largeur / longueur de substrat inférieure à 3 cm et peut ainsi aisément être intégrée dans un dispositif biotélémétrique telle qu'une capsule biotélémétrique. Cette antenne est une antenne à bas profil (« low profile » selon la terminologie anglo-saxonne), l'épaisseur de l'antenne étant négligeable par rapport à sa taille. Une telle antenne occupe un espace négligeable dans une capsule biotélémétrique, permet un degré de miniaturisation élevé tout en ayant une efficacité accrue en transmission.

Une telle antenne radioélectrique peut en outre être réalisée sur un substrat flexible, de sorte à pouvoir se conformer à la surface intérieure d'un dispositif biotélémétrique tel qu'une capsule.

Dans un ou plusieurs modes de réalisation de l'antenne radioélectrique selon le premier aspect, le segment rectiligne est orienté selon une première direction et la bande en matériau conducteur s'étend longitudinalement selon une deuxième direction distincte de la première direction.

Dans un ou plusieurs modes de réalisation de l'antenne radioélectrique selon le premier aspect, la première direction est sensiblement perpendiculaire à la deuxième direction.

Dans un ou plusieurs modes de réalisation de l'antenne radioélectrique selon le premier aspect, la bande de matériau conducteur est de forme parallélépipédique, en serpentin ou en zigzag.

Dans un ou plusieurs modes de réalisation de l'antenne radioélectrique selon le premier aspect, le segment rectiligne est orienté selon une première direction et la bande en matériau conducteur s'étend longitudinalement selon une deuxième direction distincte de la première direction, dans laquelle la fréquence fres de résonance de l'antenne radioélectrique est liée à la dimension lhigh selon la première direction du segment rectiligne et la dimension llow selon la première direction de la bande de matériau conducteur, à l'impédance caractéristique Zc-low du premier élément et à l'impédance caractéristique Zc-high du deuxième élément par les relations :

$$- Z_{\text{c-low}} + Z_{\text{c-high}} \tan(\beta_{\text{high}} \, l_{\text{high}}) \tan(\beta_{\text{low}} \, l_{\text{low}}) = 0$$

$$Z_{\text{c-high}} \tan(\beta_{\text{low}} \, l_{\text{low}}) + Z_{\text{c-low}} \tan(\beta_{\text{high}} \, l_{\text{high}}) \neq 0$$

$\beta_{\text{low}}$ étant la constante de phase du premier élément définie par :

$$\beta_{low} = \frac{2\pi}{c} f_{res} \sqrt{\varepsilon_{low}^{r,eff}}$$

où $\beta_{\text{high}}$ est la constante de phase du deuxième élément définie par

$$\beta_{high} = \frac{2\pi}{c} f_{res} \sqrt{\varepsilon_{high}^{r,eff}}$$

$c$ étant la vitesse de la lumière,

$\varepsilon_{high}^{r,eff}$ étant la permittivité relative effective du deuxième élément,

$\varepsilon_{low}^{r,eff}$ étant la permittivité relative effective du premier élément.

Dans un ou plusieurs modes de réalisation de l'antenne radioélectrique selon le premier aspect, le rapport entre la largeur maximale de la bande en matériau conducteur et la largeur du segment rectiligne est compris entre 5 et 0,2.

Les caractéristiques des différents modes de réalisation de l'antenne radioélectrique selon le premier aspect sont combinables entre elles.

La présente description a pour objet, selon un deuxième aspect, un dispositif biotélémétrique comprenant une antenne radioélectrique selon le premier aspect. Les caractéristiques, propriétés, avantages et/ou effets de l'antenne radioélectrique selon le premier aspect sont transposables directement au dispositif biotélémétrique selon le deuxième aspect.

Dans un ou plusieurs modes de réalisation du dispositif biotélémétrique selon le deuxième aspect, le substrat étant dans un matériau flexible et le dispositif biotélémétrique étant sous forme de capsule dans laquelle le substrat est roulé de sorte que la première face du substrat est tournée vers l'intérieur de la capsule et la deuxième face est tournée vers l'extérieur de la capsule.

Dans un ou plusieurs modes de réalisation du dispositif biotélémétrique selon le deuxième aspect, le substrat de l'antenne radioélectrique est un substrat polyimide flexible se conformant à la surface interne de la capsule.

Dans un ou plusieurs modes de réalisation du dispositif biotélémétrique selon le deuxième aspect, le substrat est dans un matériau rigide et de forme cylindrique, le dispositif biotélémétrique étant intégré dans une capsule dans laquelle l'antenne radioélectrique est placée que la première face du substrat soit tournée vers l'intérieur de la capsule et la deuxième face soit tournée vers l'extérieur de la capsule.

L'invention porte aussi sur l'utilisation d'une antenne radioélectrique selon l'un quelconque des modes de réalisation ci-dessus dans un milieu environnant, l'antenne étant placée de sorte que la deuxième face sur laquelle est arrangé le premier élément soit placée du côté du milieu environnant l'antenne et que le deuxième élément soit du côté opposé au milieu environnant.

BREVE DESCRIPTION DES FIGS.

D'autres avantages et caractéristiques de la technique présentée ci-dessus apparaîtront à la lecture de la description détaillée ci-dessous, faite par référence aux FIGS. dans lesquelles :

- la FIG. 1 représente de manière schématique un dispositif biotélémétrique selon un exemple de réalisation ;
- les FIG. 2A et 2B représentent de manière schématique une antenne radioélectrique selon un exemple de réalisation ;
- la FIG. 3 représente de manière schématique un dispositif biotélémétrique selon un exemple de réalisation ;
- la FIG. 4 illustre des aspects du fonctionnement et des performances d'une antenne radioélectrique selon un exemple de réalisation.

Dans les différents modes de réalisation qui vont être décrits par référence aux FIGS., des éléments semblables ou identiques portent les mêmes références.

DESCRIPTION DETAILLEE

Les différents modes de réalisation et aspects décrits ci-dessous peuvent être combinés ou simplifiés de multiples manières. Seuls certains modes de réalisation d'exemples sont décrits en détail pour assurer la clarté de l'exposé mais ces exemples ne visent pas à limiter la portée générale des principes ressortant de cette description considérée dans son ensemble.

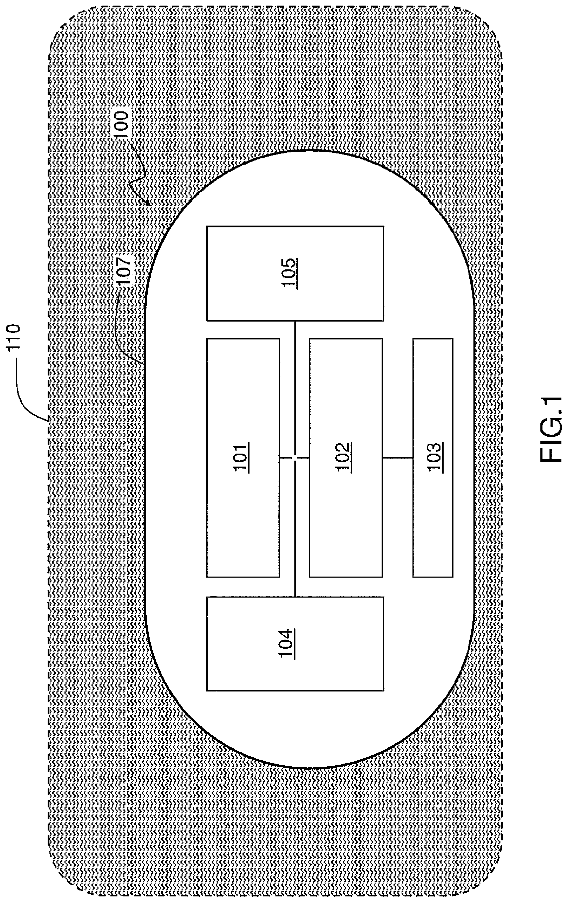

La FIG. 1 représente de manière schématique un exemple de dispositif biotélémétrique 100, sous forme de capsule ingestible.

Le dispositif biotélémétrique 100 comprend un microcontrôleur 101, un circuit radiofréquence 102, une antenne radioélectrique 103, une source d'alimentation 104. De manière optionnelle, le dispositif biotélémétrique 100 peut comprendre un circuit additionnel 105, par exemple un circuit d'application biomédicale ou un capteur.

Dans un ou plusieurs modes de réalisation, la source d'alimentation 104 est configurée pour alimenter électriquement le microcontrôleur 101, le circuit radiofréquence 102, l'antenne radioélectrique 103 et le circuit additionnel 105.

Dans un ou plusieurs modes de réalisation, l'antenne radioélectrique 103 est configurée pour communiquer via une liaison radio avec un dispositif externe (non représenté), l'antenne radioélectrique 103 peut par exemple transmettre des données (par exemple des données de biotélémétrie acquises par le dispositif biotélémétrique 100) vers le dispositif externe et recevoir des données (par exemple des instructions opérationnelles et/ou de traitement thérapeutique) en provenance d'un tel dispositif externe.

Dans un ou plusieurs modes de réalisation, l'antenne radioélectrique peut émettre et recevoir des ondes électromagnétiques à fréquences élevées, par exemple dans la gamme de $10^8$ Hz à $10^{10}$ Hz.

Selon un ou plusieurs modes de réalisation, le microcontrôleur comprend une unité 112 de génération de signal électrique configurée pour générer le signal électrique incident. Selon un ou plusieurs modes de réalisation, le microcontrôleur comprend une unité 113 de traitement de données.

Dans un ou plusieurs modes de réalisation, le microcontrôleur 101 est configuré pour générer un signal électrique incident à convertir en onde électromagnétique par l'antenne radioélectrique et/ou pour amplifier un signal reçu en provenance de l'antenne radioélectrique.

Dans un ou plusieurs modes de réalisation, le microcontrôleur 101 est configuré pour traiter des données, par exemple pour traiter les données reçues par l'antenne radioélectrique 103 ou des données acquises par le circuit additionnel 105.

Dans un ou plusieurs modes de réalisation, l'ensemble des composants du dispositif biotélémétrique 100 (le microcontrôleur 101, le circuit radiofréquence 102, l'antenne radioélectrique 103, la source d'alimentation 104 et de manière optionnelle, le un circuit additionnel 105) est intégré dans une capsule biocompatible 107.

Dans un ou plusieurs modes de réalisation, le circuit radiofréquence 102 est interconnecté entre le microcontrôleur 101 et l'antenne radioélectrique 103. Le circuit radiofréquence 102 sert d'interface électrique entre le microcontrôleur 101.

Dans un ou plusieurs modes de réalisation, le circuit d'application biomédicale 105 est configuré pour mettre en oeuvre des fonctions de diagnostic et/ou des fonctions thérapeutiques. Les fonctions de diagnostic peuvent comprendre des fonctions d'acquisition ou mesure de données de diagnostic, par exemple au moyen d'un ou plusieurs capteurs, tels que par exemple, capteurs de température, capteurs électroniques, MEMS (« Microelectromechanical systems ») ou capteurs microfluidiques. Les fonctions de diagnostic peuvent comprendre des fonctions d'endoscopie, d'acquisition d'images, de mesure de glucose ou d'autres paramètres physiologiques, de détection d'anticorps, etc. Les fonctions thérapeutiques peuvent comprendre par exemple la délivrance de médicaments et la stimulation électrique, par exemple la stimulation nerveuse.

Le dispositif biotélémétrique 100 est destiné à être utilisé dans un milieu environnant 110, par exemple après ingestion ou implantation *in vivo*. Au fur et à mesure que le dispositif biotélémétrique 100 se déplace dans le corps humain, par exemple pendant le transit gastro-intestinal, ce milieu environnant 110 est susceptible d'avoir des propriétés variées.

Les propriétés électromagnétiques (EM) du milieu environnant 110 entourant le dispositif biotélémétrique 100 détermine le couplage entre l'antenne radioélectrique 103 et le milieu environnant 110 et l'absorption des champs EM par ce milieu environnant 110. La connaissance de ces propriétés EM permet d'adapter la configuration de l'antenne radioélectrique 103 pour optimiser les performances de transmission sans fil de l'antenne radioélectrique 103 à travers le milieu environnant. En particulier, lorsque le couplage entre l'antenne radioélectrique 103 et le milieu environnant 110 est important, et les propriétés en transmission de l'antenne radioélectrique peuvent être affectées par les variations des propriétés EM du milieu environnant 110 dans lequel le dispositif biotélémétrique 100 se situe.

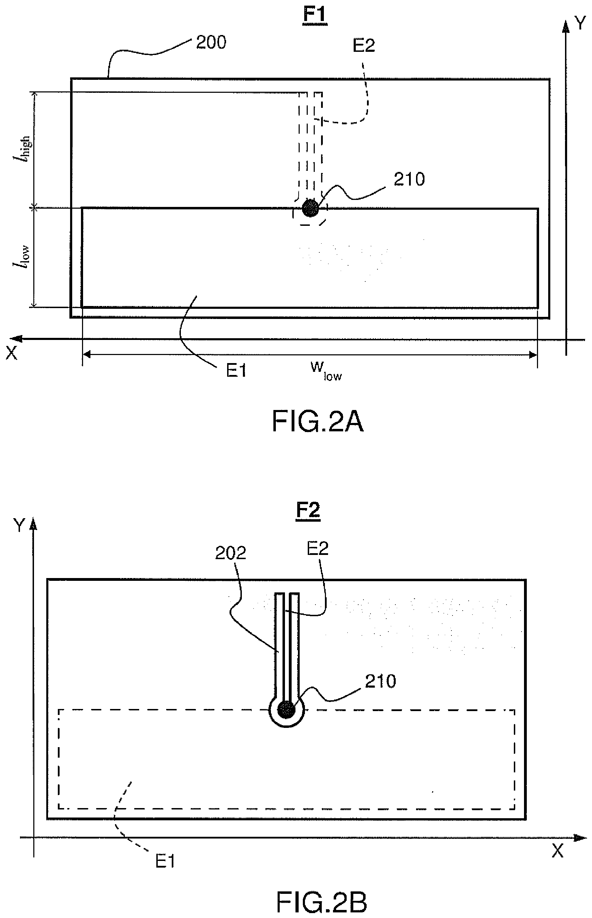

La FIG. 2 représente de manière schématique une configuration géométrique d'une antenne radioélectrique 200 adaptée pour un dispositif biotélémétrique 100 tel que celui décrit par référence à la FIG. 1, selon un exemple de réalisation.

Dans un ou plusieurs modes de réalisation, l'antenne radioélectrique 200 comprend un substrat 210 formé en un matériau diélectrique (FR4, PFTE, polyimide, polyétheréthercétone, céramiques, composites etc.). Le substrat 210 est constitué soit d'un matériau souple et/ou pouvant être roulé, de sorte à pouvoir se conformer à la surface intérieure d'un dispositif biotélémétrique tel qu'une capsule, soit d'un matériau rigide convenant pour la réalisation d'un dispositif biotélémétrique planaire. Le substrat est par exemple en polyimide flexible : un tel matériau est apte à se conformer à la surface interne d'une capsule de dispositif biotélémétrique.

Dans un ou plusieurs modes de réalisation, un plan de masse électriquement conducteur est agencé sur une première face F2 (dessous) du substrat. Le plan de masse est par exemple réalisé en matériau électriquement conducteur (par exemple, en métal tel que cuivre, aluminium, argent, etc. ou un alliage).

Dans un ou plusieurs modes de réalisation, l'antenne radioélectrique 200 comprend un résonateur comprenant un premier élément E1, ayant une première impédance caractéristique Zc-low, et un deuxième élément E2, ayant une deuxième impédance caractéristique Zc-high.

Dans un ou plusieurs modes de réalisation, le résonateur est configuré pour convertir un signal électrique incident produit par le microcontrôleur en une onde électromagnétique à une fréquence de résonance dépendant des caractéristiques structurelles du résonateur. La fréquence de résonance est la fréquence pour laquelle la partie imaginaire de l'impédance de l'antenne égale à zéro : $Im(Z_{ANT}) = 0$.

Dans un ou plusieurs modes de réalisation, le premier élément E1 est configuré pour recevoir le signal électrique incident généré par le microcontrôleur. Un point d'alimentation électrique est prévu en un endroit arbitraire du premier élément. L'alimentation électrique peut être effectuée par microruban, un câble coaxial, ou par un couplage en proximité.

Dans un ou plusieurs modes de réalisation, le premier élément E1 est formé par une bande en matériau électriquement conducteur, la bande étant agencée sur une deuxième face F1 (dessus) du substrat opposée à la première face F2. Le matériau du plan de masse peut être identique ou différent du matériau de la bande en matériau conducteur, (par exemple un métal tel que cuivre, aluminium, argent, etc. ou un alliage) La bande de matériau conducteur peut avoir différentes formes géométriques : une forme parallélépipédique, par exemple rectangulaire comme dans le cas d'exemple représenté à la FIG. 2, une forme en serpentin ou une forme en zigzag, etc

Dans un ou plusieurs modes de réalisation, le deuxième élément E2 est formé par un segment rectiligne, découpé dans le plan de masse, et séparé du reste du plan de masse par une fente 202 de largeur fixe ou sensiblement fixe, aux tolérances de réalisation près.

Dans un ou plusieurs modes de réalisation, le deuxième élément E2 est relié électriquement au plan de masse au niveau d'une première extrémité du segment rectiligne. Dans un ou plusieurs modes de réalisation, le deuxième élément E2 est relié électriquement au premier élément E1 au niveau d'une deuxième extrémité du segment rectiligne au moyen d'un via 210 traversant le substrat. Le premier élément E1 est ainsi relié électriquement au plan de masse par l'intermédiaire du via 210 et du deuxième élément E2.

Dans un ou plusieurs modes de réalisation, le via est relié à un bord de la bande de matériau conducteur. Dans un ou plusieurs modes de réalisation, la fente 202 entoure le via 210 et isole le via 210 du plan de masse à l'exception de l'extrémité du via 210 qui est connectée à la deuxième extrémité du segment rectiligne.

Dans un ou plusieurs modes de réalisation, le résonateur est un résonateur à saut d'impédance (SIR, pour « stepped impédance resonator ») à quart d'onde, avec une transition de basse impédance à haute impédance et un court-circuit au plan de masse à l'extrémité de l'élément à plus haute impédance (extrémité haute impédance). Ceci implique une distribution de courant électrique présentant son minimum à l'extrémité de l'élément à plus basse impédance (extrémité basse impédance) et son maximum à l'extrémité de l'élément à plus haute impédance (extrémité haute impédance). La distribution de tension est inverse de celle de courant.

Dans un ou plusieurs modes de réalisation, le rapport entre la dimension maximale selon l'axe Y $l_{tow}$ de la bande de matériau conducteur et la dimension $l_{high}$ selon l'axe Y du segment rectiligne est compris entre 5 et 0,2. La longueur totale de l'antenne ($l_{low} + l_{high}$) augmentant avec la dimension maximale selon l'axe Y $l_{low}$ de la bande de

EP 3 692 595 B1

matériau conducteur, cela réduit le facteur de miniaturisation de l'antenne.

Dans un ou plusieurs modes de réalisation, le segment rectiligne s'étend longitudinalement selon une première direction (axe Y dans la FIG. 2) et la bande en matériau conducteur s'étend longitudinalement selon une deuxième direction (axe X dans la FIG. 2), distincte de la première direction. Dans un ou plusieurs modes de réalisation, correspondant à l'exemple illustré à la FIG. 2, la première direction est perpendiculaire à la deuxième direction ou sensiblement perpendiculaire à la deuxième direction. Dans un ou plusieurs modes de réalisation, la première direction est fait un angle compris entre 0° et 45° avec la deuxième direction.

Dans un ou plusieurs modes de réalisation, l'impédance caractéristique Zc-high du deuxième élément E2 est supérieure à la l'impédance caractéristique Zc-low du premier élément E1. En pratique, l'impédance caractéristique Zc-high du deuxième élément E2 dépend de la largeur du segment rectiligne, de la distance du segment rectiligne au plan de masse, c'est-à-dire de la largeur de la fente 202 et des propriétés électromagnétiques et géométriques des matériaux environnants.

La géométrie de l'antenne radioélectrique satisfaisant la condition de résonance, i.e. (la fréquence de résonance correspond à fréquence pour laquelle la partie imaginaire de l'impédance de l'antenne est nulle : $\text{Im}(Z_{ANT}) = 0$) peut être déduite de l'équation d'impédance des lignes de transmission.

Dans le cas de configuration géométrique décrite par référence à la FIG. 2, la fréquence de résonance $f_{res}$ de l'antenne radioélectrique est liée à la dimension $l_{high}$ selon l'axe Y du segment rectiligne et à la dimension selon l'axe Y $l_{low}$ de la bande de matériau conducteur, à l'impédance caractéristique Zc-low du premier élément et à l'impédance caractéristique Zc-high du deuxième élément par les relations suivantes :

$$- Z_{\text{c-low}} + Z_{\text{c-high}} \tan(\beta_{\text{high}} l_{\text{high}}) \tan(\beta_{\text{low}} l_{\text{low}}) = 0 \qquad (eq1a)$$

$$Z_{\text{c-high}} \tan(\beta_{\text{low}} l_{\text{low}}) + Z_{\text{c-low}} \tan(\beta_{\text{high}} l_{\text{high}}) \neq 0 \qquad (eq1b)$$

$\beta_{\text{low}}$ étant la constante de phase du premier élément définie par :

$$\beta_{low} = \frac{2\pi}{c} f_{res} \sqrt{\varepsilon_{low}^{r,eff}}$$

où $\beta_{\text{high}}$ est la constante de phase du deuxième élément définie par

$$\beta_{high} = \frac{2\pi}{c} f_{res} \sqrt{\varepsilon_{high}^{r,eff}}$$

c étant la vitesse de la lumière,

$\varepsilon_{high}^{r,eff}$ étant la permittivité relative effective du deuxième élément,

$\varepsilon_{low}^{r,eff}$ étant la permittivité relative effective du premier élément.

Pour concevoir une antenne présentant une fréquence de résonance $f_{res}$ donnée, on peut appliquer la procédure suivante. On choisit la dimension totale ($l_{high} + l_{low}$) selon l'axe Y de l'antenne radiofréquence de l'antenne radioélectrique, par exemple en fonction du dispositif biotélémétrique cible. On choisit ensuite la largeur utile maximale $w_{low}$ de l'élément E1 ayant la plus basse impédance (bande de matériau). Puis, on détermine l'impédance caractéristique $Z_{\text{c-low}}$ du premier élément et à l'impédance caractéristique $Z_{\text{c-high}}$ du deuxième élément ainsi que la permittivité relative effective du premier élément et la permittivité relative effective du deuxième élément en résolvant les équations (eq1a) et (eq1b) ci-dessus par une méthode numérique formulée en 2D. Les dimensions précises des éléments E1 et E2 peuvent ensuite être finement ajustées à l'aide d'une méthode numérique formulée en 3D.

On peut par exemple réaliser une antenne à 434 MHz $l_{high}$ = 4.3 mm, $l_{low}$ = 4.6 mm, $w_{low}$ = 20 mm et une largeur de fente 202 de 180 μm.

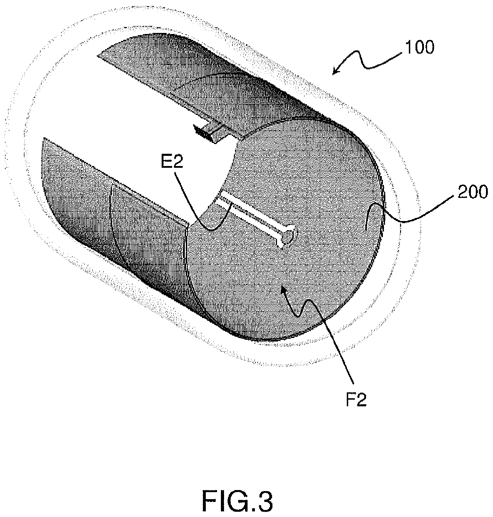

La FIG. 3 représente de manière schématique un dispositif biotélémétrique 100 comprenant une antenne radioélectrique 200 selon la présente description dans un exemple de réalisation.

Le dispositif biotélémétrique est sous forme de capsule dans laquelle le substrat est intégré de sorte que la face F2 du substrat sur laquelle est agencé le plan de masse et le segment rectiligne E2 soit tournée vers l'intérieur de

7

la capsule et que l'autre face F1 sur laquelle est agencé le premier élément E1 soit tournée vers l'extérieur de la capsule. La taille de la capsule est par exemple de 17,7 mm de longueur et de 8,9 mm de diamètre.

Dans un ou plusieurs modes de réalisation, le substrat de l'antenne radioélectrique est un substrat en matériau flexible, par exemple en polyimide flexible de 50 μm d'épaisseur, se conformant à la surface interne de la capsule.

Dans un ou plusieurs modes de réalisation, le substrat est dans un matériau rigide et de forme cylindrique de sorte à former une antenne radioélectrique 200 cylindrique. Les dimensions du substrat et le diamètre du cylindre formé par le substrat sont dans ce cas adaptés aux dimensions internes de la capsule avec une tolérance de 50 μm.

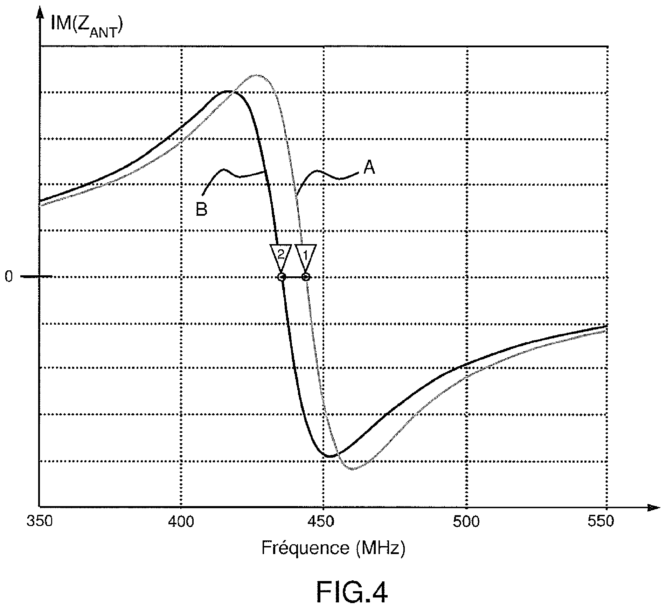

La FIG. 4 montre les courbes de variation de la partie imaginaire de l'impédance (Im($Z_{ANT}$)) de l'antenne radioélectrique conçue pour la bande ISM (industriel, scientifique et médical) 434 MHz en fonction de la fréquence dans un exemple de réalisation. L'antenne radioélectrique a été réalisée sur un circuit imprimé.

Dans cet exemple de réalisation, $l_{high}$ = 4.3 mm, $l_{low}$ = 4.6 mm, $w_{low}$ = 20 mm et une largeur de fente 202 de 180 μm. La fréquence de fonctionnement est de 434 MHz.

La courbe A (la plus à droite) correspond aux variations d'impédance dans l'air, tandis que la courbe B (la plus à gauche) correspond aux variations d'impédance dans un milieu fantôme équivalent à un muscle de perméabilité relative 56,9, de conductivité 0,81 S/m à la fréquence de 434 MHz.

Le point de la courbe pour lequel la partie imaginaire de l'impédance est nulle donne la fréquence de résonance de l'antenne radioélectrique. Dans l'air (courbe A), la fréquence de résonance obtenue est de 443,8 MHz. Dans le fantôme de muscle (courbe B), la fréquence de résonance obtenue est de 435,1 MHz.

En comparant ces fréquences de résonance, on constate ainsi que la fréquence de résonance ne change que de 2% environ, ce qui montre la très faible sensibilité de l'impédance de l'antenne radioélectrique aux changements d'environnement. Ce changement est très faible par rapport à d'autres antennes radioélectriques présentant des taux de variation de la fréquence de résonance de plus de 600%, comme par exemple celle décrite dans le document intitulé « Outer-wall loop antenna for ultrawideband capsule endoscope system » de Yun. S. et al., IEEE Antennas Wireless Propagation Letters, vol. 9 pages 1135-1138, 2010, pour laquelle la fréquence de résonance obtenue dans l'air est de 2,7 GHz et de 434 MHz pour le fantôme de muscle

L'antenne radioélectrique décrite dans ce document peut être conçue pour fonctionner dans une large gamme de fréquence de résonance, par exemple entre $10^8$ Hz et $5 \times 10^9$ Hz.

L'antenne radioélectrique décrite dans ce document présente de nombreuses possibilités d'application que ce soit dans le domaine médical ou non médical, par exemple, pour le génie civil, l'agriculture, la transformation des aliments, etc. L'antenne radioélectrique est utilisable quand une forte robustesse en impédance est requise en raison des variations de l'environnement dans lequel cette antenne radioélectrique est destinée à être utilisée (par exemple, le corps humain et/ou animal, mais aussi l'air, l'eau, les vêtements, la pluie, la sueur, etc.

Une application est son utilisation dans un dispositif biotélémétrique ingestible et/ou implantable *in vivo* pour des applications de biotélémétrie et de téléthérapie dans le corps humain et/ou animal. Une autre application émergente est la télésurveillance de modèles de corps artificiels et prothèses.

**Revendications**

1. Antenne radioélectrique, l'antenne radioélectrique comprenant

   un substrat formé en un matériau diélectrique ;
   un plan de masse en matériau électriquement conducteur, le plan de masse étant agencé sur une première face (F2) du substrat ;
   un résonateur configuré pour convertir un signal électrique incident en une onde électromagnétique, le résonateur comprenant un premier élément (E1) ayant une première impédance caractéristique et un deuxième élément (E2) ayant une deuxième impédance caractéristique supérieure à la première impédance caractéristique;
   dans laquelle
   le premier élément (E1) est configuré pour recevoir le signal électrique incident, le premier élément (E1) est formé par une bande en matériau électriquement conducteur, la bande étant agencée sur une deuxième face (F1) du substrat opposée à la première face (F2) ;
   le deuxième élément (E2) est formé par un segment rectiligne, découpé dans le plan de masse et séparé du reste du plan de masse par une fente (202) de largeur fixe, le deuxième élément (E2) est relié électriquement au plan de masse au niveau d'une première extrémité du segment et relié électriquement au premier élément au niveau d'une deuxième extrémité du segment au moyen d'un via (210) traversant le substrat,
   l'antenne radioélectrique étant configurée de sorte que la deuxième face (F1) sur laquelle est arrangé le premier élément (E1) soit placée du côté d'un milieu environnant l'antenne et que le deuxième élément (E2) soit du côté opposé au milieu environnant.

**2.** Antenne radioélectrique selon la revendication 1, dans laquelle le segment rectiligne est orienté selon une première direction (Y) et la bande en matériau conducteur s'étend longitudinalement selon une deuxième direction (X) distincte de la première direction.

**3.** Antenne radioélectrique selon la revendication 2, dans laquelle la première direction est sensiblement perpendiculaire à la deuxième direction.

**4.** Antenne radioélectrique selon l'une quelconque des revendications précédentes, dans laquelle la bande de matériau conducteur est de forme parallélépipédique, en serpentin ou en zigzag.

**5.** Antenne radioélectrique selon l'une quelconque des revendications précédentes, dans laquelle le segment rectiligne est orienté selon une première direction (Y) et la bande en matériau conducteur s'étend longitudinalement selon une deuxième direction (X) distincte de la première direction,

dans laquelle la fréquence $f_{res}$ de résonance de l'antenne radioélectrique est liée à la dimension $l_{high}$ selon la première direction (Y) du segment rectiligne et la dimension $l_{low}$ selon la première direction (Y) de la bande de matériau conducteur, à l'impédance caractéristique $Z_{c\text{-}low}$ du premier élément et à l'impédance caractéristique $Z_{c\text{-}high}$ du deuxième élément par les relations :

$$- Z_{c\text{-}low} + Z_{c\text{-}high} \tan\left(\beta_{high}\, l_{high}\right) \tan(\beta_{low}\, l_{low}) = 0$$

$$Z_{c\text{-}high} \tan(\beta_{low}\, l_{low}) + Z_{c\text{-}low} \tan(\beta_{high}\, l_{high}) \neq 0$$

$\beta_{low}$ étant la constante de phase du premier élément définie par :

$$\beta_{low} = \frac{2\pi}{c} f_{res} \sqrt{\varepsilon_{low}^{r,eff}}$$

où $\beta_{high}$ est la constante de phase du deuxième élément définie par

$$\beta_{high} = \frac{2\pi}{c} f_{res} \sqrt{\varepsilon_{high}^{r,eff}}$$

$c$ étant la vitesse de la lumière,

$\varepsilon_{high}^{r,eff}$ étant la permittivité relative effective du deuxième élément,

$\varepsilon_{low}^{r,eff}$ étant la permittivité relative effective du premier élément.

**6.** Antenne radioélectrique selon l'une quelconque des revendications précédentes dans laquelle le rapport entre la largeur maximale de la bande en matériau conducteur et la largeur du segment rectiligne est compris entre 5 et 0,2.

**7.** Dispositif biotélémétrique comprenant une antenne radioélectrique selon l'une quelconque des revendications précédentes.

**8.** Dispositif biotélémétrique selon la revendication 7, le substrat étant dans un matériau flexible et le dispositif biotélémétrique étant sous forme de capsule dans laquelle le substrat est roulé de sorte que la première face (F2) du substrat est tournée vers l'intérieur de la capsule et la deuxième face (F1) est tournée vers l'extérieur de la capsule.

**9.** Dispositif biotélémétrique selon la revendication 8, dans lequel le substrat de l'antenne radioélectrique est un substrat polyimide flexible se conformant à la surface interne de la capsule.

**10.** Dispositif biotélémétrique selon la revendication 7, dans lequel le substrat est dans un matériau rigide et de forme cylindrique, le dispositif biotélémétrique étant intégré dans une capsule dans laquelle l'antenne radioélectrique est placée que la première face du substrat soit tournée vers l'intérieur de la capsule et la deuxième face soit tournée vers l'extérieur de la capsule.

**11.** Utilisation d'une antenne radioélectrique selon l'une quelconque des revendications 1 à 6 ou d'un dispositif biotélémétrique suivant l'une quelconque des revendications 7 à 10 dans un milieu environnant, l'antenne étant placée de sorte que la deuxième face (F1) sur laquelle est arrangé le premier élément (E1) soit placée du côté du milieu environnant l'antenne et que le deuxième élément (E2) soit du côté opposé au milieu environnant.

**Patentansprüche**

**1.** Funkantenne, wobei die Funkantenne Folgendes umfasst:

ein aus einem dielektrischen Material gebildetes Substrat;
eine Masseebene aus einem elektrisch leitfähigen Material, wobei die Masseebene auf einer ersten Seite (F2) des Substrats angeordnet ist;
einen Resonator, der zum Umwandeln eines einfallenden elektrischen Signals in eine elektromagnetische Welle konfiguriert ist, wobei der Resonator ein erstes Element (E1) mit einer ersten charakteristischen Impedanz und ein zweites Element (E2) mit einer zweiten charakteristischen Impedanz umfasst, die größer als die erste charakteristische Impedanz ist;
wobei
das erste Element (E1) zum Empfangen des einfallenden elektrischen Signals konfiguriert ist, das erste Element (E1) durch einen Streifen aus elektrisch leitfähigem Material gebildet wird, wobei der Streifen auf einer zweiten Seite (F1) des Substrats gegenüber der ersten Seite (F2) angeordnet ist;
das zweite Element (E2) durch ein geradliniges Segment gebildet wird, das in die Masseebene geschnitten und vom Rest der Masseebene durch einen Schlitz (202) mit fester Breite getrennt ist, wobei das zweite Element (E2) an einem ersten Ende des Segments elektrisch mit der Masseebene verbunden ist und an einem zweiten Ende des Segments mittels eines das Substrat durchquerenden Durchkontaktierung (210) elektrisch mit dem ersten Element verbunden ist,
wobei die Funkantenne so konfiguriert ist, dass die zweite Seite (F1), auf der das erste Element (E1) angeordnet ist, auf der Seite eines die Antenne umgebenden Mediums platziert ist und dass das zweite Element (E2) auf der dem umgebenden Medium gegenüberliegenden Seite liegt.

**2.** Funkantenne nach Anspruch 1, wobei das geradlinige Segment in einer ersten Richtung (Y) orientiert ist und der Streifen aus leitfähigem Material sich longitudinal in einer zweiten Richtung (X) erstreckt, die sich von der ersten Richtung unterscheidet.

**3.** Funkantenne nach Anspruch 2, wobei die erste Richtung im Wesentlichen lotrecht zur zweiten Richtung verläuft.

**4.** Funkantenne nach einem der vorherigen Ansprüche, wobei der Streifen aus leitfähigem Material parallelepipedförmig, schlangenförmig oder zickzackförmig ist.

**5.** Funkantenne nach einem der vorherigen Ansprüche, wobei das geradlinige Segment in einer ersten Richtung (Y) orientiert ist und der Streifen aus leitfähigem Material sich longitudinal in einer zweiten Richtung (X) erstreckt, die sich von der ersten Richtung unterscheidet,

wobei die Resonanzfrequenz $f_{res}$ der Funkantenne mit der Abmessung $l_{high}$ in der ersten Richtung (Y) des geradlinigen Segments und der Abmessung $l_{low}$ in der ersten Richtung (Y) des Streifens aus leitfähigem Material, mit der charakteristischen Impedanz $Z_{c-low}$ des ersten Elements und mit der charakteristischen Impedanz $Z_{c-high}$ des zweiten Elements durch die folgenden Beziehungen verknüpft ist:

$$- Z_{c-low} + Z_{c-high} \tan(\beta_{high}\, l_{high}) \tan(\beta_{low}\, l_{low}) = 0$$

$$Z_{c-high} \tan(\beta_{low}\, l_{low}) + Z_{c-low} \tan(\beta_{high}\, l_{high}) \neq 0$$

wobei $\beta_{\text{low}}$ die Phasenkonstante des ersten Elements ist, definiert durch:

$$\beta_{\text{low}} = \frac{2\pi}{c} f_{res} \sqrt{\varepsilon_{low}^{r,eff}}$$

wobei $\beta_{\text{high}}$ die Phasenkonstante des zweiten Elements ist, definiert durch

$$\beta_{\text{high}} = \frac{2\pi}{c} f_{res} \sqrt{\varepsilon_{high}^{r,eff}}$$

wobei c die Lichtgeschwindigkeit ist,

wobei $\varepsilon_{high}^{r,eff}$ die effektive relative Permittivität des zweiten Elements ist,

wobei $\varepsilon_{low}^{r,eff}$ die effektive relative Permittivität des ersten Elements ist.

6. Funkantenne nach einem der vorherigen Ansprüche, wobei das Verhältnis zwischen der maximalen Breite des Streifens aus leitfähigem Material und der Breite des geradlinigen Segments zwischen 5 und 0,2 liegt.

7. Biotelemetrische Vorrichtung mit einer Funkantenne nach einem der vorherigen Ansprüche.

8. Biotelemetrische Vorrichtung nach Anspruch 7, wobei das Substrat aus einem flexiblen Material besteht und die biotelemetrische Vorrichtung die Form einer Kapsel hat, in die das Substrat gerollt ist, so dass die erste Seite (F2) des Substrats dem Inneren der Kapsel zugewandt ist und die zweite Seite (F1) dem Äußeren der Kapsel zugewandt ist.

9. Biotelemetrische Vorrichtung nach Anspruch 8, wobei das Substrat der Funkantenne ein flexibles Polyimidsubstrat ist, das sich an die Innenfläche der Kapsel anpasst.

10. Biotelemetrische Vorrichtung nach Anspruch 7, wobei das Substrat aus einem starren Material besteht und eine zylindrische Form hat, wobei die biotelemetrische Vorrichtung in eine Kapsel integriert ist, in der die Funkantenne so platziert ist, dass die erste Seite des Substrats dem Inneren der Kapsel zugewandt ist und die zweite Seite dem Äußeren der Kapsel zugewandt ist.

11. Verwendung einer Funkantenne nach einem der Ansprüche 1 bis 6 oder einer biotelemetrischen Vorrichtung nach einem der Ansprüche 7 bis 10 in einem umgebenden Medium, wobei die Antenne so platziert ist, dass die zweite Seite (F1), auf der das erste Element (E1) angeordnet ist, auf der Seite des die Antenne umgebenden Mediums platziert ist und dass das zweite Element (E2) auf der dem umgebenden Medium gegenüberliegenden Seite liegt.

## Claims

1. A radio antenna, the radio antenna comprising:

   a substrate formed of a dielectric material;
   a ground plane of electrically conductive material, the ground plane being arranged on a first face (F2) of the substrate;
   a resonator configured to convert an incident electrical signal into an electromagnetic wave, the resonator comprising a first element (E1) having a first characteristic impedance and a second element (E2) having a second characteristic impedance greater than the first characteristic impedance;
   in which
   the first element (E1) is configured to receive the incident electrical signal, the first element (E1) is formed by a strip of electrically conductive material, the strip being arranged on a second face (F1) of the substrate opposite to the first face (F2);
   the second element (E2) is formed by a rectilinear segment, cut out in the ground plane and separated from the rest of the ground plane by a slot (202) of fixed width, the second element (E2) is electrically connected to the ground plane at a first end of the segment and electrically connected to the first element at a second end of the segment by means of a via (210) passing through the substrate.

the radio antenna being configured so that the second face (F1) on which the first element (E1) is arranged is placed on the side of a medium surrounding the antenna and the second element (E2) is on the side opposite the surrounding environment.

2. Radio antenna according to claim 1, wherein the rectilinear segment is oriented in a first direction (Y) and the strip of conductive material extends longitudinally in a second direction (X) distinct from the first direction.

3. Radio antenna according to claim 2, wherein the first direction is substantially perpendicular to the second direction.

4. Radio antenna according to any one of the preceding claims, in which the strip of conductive material is of parallelepiped, serpentine or zigzag shape.

5. Radio antenna according to any one of the preceding claims, in which the rectilinear segment is oriented in a first direction (Y) and the strip of conductive material extends longitudinally in a second direction (X) distinct from the first direction,

in which the resonance frequency $f_{res}$ of the radio antenna is related to the dimension $l_{high}$ according to the first direction (Y) of the rectilinear segment and the dimension $l_{low}$ along the first direction (Y) of the strip of conductive material, to the characteristic impedance $Z_{c\_low}$ of the first element and to the characteristic impedance $Z_{c\text{-}high}$ of the second element by the relations:

$$-Z_{c\text{-}low} + Z_{c\text{-}high} \tan(\beta_{high}\, l_{high}) \tan(\beta_{low}\, l_{low}) = 0$$

$$Z_{c\text{-}high} \tan(\beta_{low}\, l_{low}) + Z_{c\text{-}low} \tan(\beta_{high}\, l_{high}) \neq 0$$

$\beta_{low}$ being the phase constant of the first element defined by:

$$\beta_{low} = (2\pi)/c\ f_{res} \sqrt{\epsilon^{r,eff}_{low}}$$

Where $\beta_{high}$ is the phase constant of the second element defined by

$$B_{high} = (2\pi)/c\ f_{res} \sqrt{\epsilon^{r,eff}_{high}}$$

c being the speed of light,

$\epsilon^{r,eff}_{high}$ being the effective relative permittivity of the second element,

$\epsilon^{r,eff}_{low}$ being the effective relative permittivity of the first element.

6. Radio antenna according to any one of the preceding claims, in which the ratio between the maximum width of the strip of conductive material and the width of the straight segment is between 5 and 0.2.

7. Biotelemetry device comprising a radio antenna according to any one of the preceding claims.

8. Biotelemetry device according to claim 7, the substrate being in a flexible material and the biotelemetry device being in the form of a capsule in which the substrate is rolled so that the first face (F2) of the substrate is turned towards the inside of the capsule and the second face (F1) faces the outside of the capsule.

9. Biotelemetry device according to claim 8, in which the substrate of the radio antenna is a flexible polyimide substrate conforming to the internal surface of the capsule.

10. Biotelemetry device according to claim 7, in which the substrate is made of a rigid material and of cylindrical shape, the biotelemetry device being integrated in a capsule wherein the radioelectric antenna is placed so that the first face of the substrate is turned towards the interior of the capsule and the second face is turned towards the outside of the capsule.

11. Use of a radio antenna according to any one of claims 1 to 6 or of a biotelemetric device according to any one of claims 7 to 10 in a surrounding environment, the antenna being placed so that the second face (F1) on which the first element (E1) is arranged is placed on the side of the environment surrounding the antenna and the second element (E2) is on the side opposite the surrounding environment.

FIG.1

EP 3 692 595 B1

**F1**

FIG.2A

**F2**

FIG.2B

FIG.3

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20170117620 A1
- KR 20140119606 A
- WO 2017086633 A
- WO 2010119207 A1

**Littérature non-brevet citée dans la description**

- **YUN. S. et al.** Outer-wall loop antenna for ultrawide-band capsule endoscope system. *IEEE Antennas Wireless Propagation Letters,* 2010, vol. 9, 1135-1138